# EUROPEAN PATENT APPLICATION

(11) **EP 3 053 518 A1**
(43) Date of publication of application: **10.08.2016**
(21) Application number: 14849996.5
(22) Date of filing: 30.09.2014
(51) Int. Cl.: A61B 5/021, A61B 5/0402, A61B 5/145, G06Q 50/22

(54) **SYSTEM FOR MONITORING BLOOD PRESSURE IN REAL-TIME**

(30) Priority: 30.09.2013 KR 20130116158; 30.09.2013 KR 20130116165
(71) Applicant: HUINNO, Co., Ltd., Gangnam-gu Seoul (KR)
(72) Inventor: GIL, Yeong Joon, Busan 612-740 (KR)
(74) Representative: Glawe, Delfs, Moll
(86) International application number: PCT/KR2014/009171
(87) International publication number: WO 2015/047015

(57) **Abstract**

A system is provided for monitoring a blood pressure in real time, including a main body and a sensor including a first electrode that is disposed on one surface of the main body and in contact with a human body when the main body is worn on the human body, a second electrode that is disposed on the other surface of the main body, and configured to measure a signal relating to at least one of electrocardiogram (ECG), photoplethysmogram (PPG) and oxygen saturation (Sp02) of the human body in contact with the first electrode and the second electrode.

## Description

### [Technical Field]

The present invention relates to a system for monitoring a blood pressure in real time, and more particularly to a system comprising a sensor module including a main body, a first electrode which is disposed on one surface of the main body and in contact with a human body when the main body is worn on the human body, and a second electrode which is disposed on the other surface of the main body to measure a signal relating to at least one of electrocardiogram (ECG), photoplethysmogram (PPG) and oxygen saturation (SpO₂) of the human body in contact with the first electrode and the second electrode.

### [Background Art]

Recently, with ongoing development of science and technology, the quality of life of the human race has been improved and many changes have occurred in the medical environment. In the past, after capturing medical images such as X-ray, Computerized Tomography (CT), and functional Magnetic Resonance Imaging (fMRI) images in the hospital, it was possible to read the images after waiting for a few hours or a few days.

However, a Picture Archiving and Communication System (PACS) has been introduced in which, after taking a medical image, the image is sent to a monitor screen of a radiology professional and can be read immediately. Further, ubiquitous healthcare/medical equipment has been adopted widely so that blood sugar and blood pressure of a patient can be checked anywhere and at any time, even without going to a hospital. Thus, patients with high blood sugar or high blood pressure are using equipment to monitor their conditions in their own homes or offices.

In the case of hypertension, which is a major cause of various diseases and whose prevalence rate is increasing, a system for continuously measuring blood pressure and displaying the measured blood pressure in real time would be desirable, and various attempts relating to this technology have been made.

As an example, ubiquitous healthcare (u_health) has been introduced in which, after measuring blood pressure in real time by inserting a blood pressure measurement sensor into a pulmonary artery of a chronic heart disease patient, a doctor may monitor changes in pulmonary artery blood pressure of the patient at a remote location when the blood pressure is transmitted to the doctor through wireless communication. The doctor may then deliver the prescription to the patient. While this approach may reduce the number of times patients go to a hospital and may provide consistent and accurate measurements of blood pressure, the approach also requires an invasive blood pressure measurement method. The approach thus also causes difficult treatment and a risk of arterial injury or infection.

Thus, a system capable of measuring blood pressure in real time by using a non-invasive method without inserting a sensor for blood pressure measurement in an artery blood vessel is desirable. Further, monitoring a blood pressure in the ubiquitous environment and performing biofeedback to provide a user with the measured blood pressure such that the user may adjust the blood pressure are also desirable.

Meanwhile, using a method of applying a cuff to an arm to measure a blood pressure has been used. However, in the case of using a cuff, it is difficult to continuously measure blood pressure because a user needs to measure the blood pressure of a patient while working or relaxing, or the patient would need to use a blood pressure meter.

In particular, informing a patient of a risk of high blood pressure rapidly so that the patient may timely receive any necessary medical attention is still needed. Thus, technology for continuously measuring blood pressure and reporting blood pressure measurement results in real time so that a patient himself or herself may prevent and manage hypertension is needed.

### [Disclosure]

### [Technical Problem]

The present invention may relate to systems and apparatuses for monitoring a blood pressure in real time, including a main body, a sensor including a first electrode that is disposed on one surface of the main body and in contact with a human body when the main body is worn on the human body, and a second electrode that is disposed on the other surface of the main body to measure a signal relating to at least one of an electrocardiogram (ECG), a photoplethysmogram (PPG), and an oxygen saturation (Sp02) of the human body in contact with the first electrode and the second electrode. A measurement processor and a monitor are included in a wearable main body such that blood pressure may be monitored continuously by applying a non-invasive method in a ubiquitous environment. A bio-signal measured by the sensor is transmitted to the Internet by using Internet Protocol version 4 (IPv4) or Internet Protocol version 6 (IPv6)-based Transmission Control Protocol/Internet Protocol (TCP/IP) communication.

The present invention may also relate to a wearable real-time blood pressure monitoring system having a main body incorporating all of a sensor measuring bio-signals and wirelessly transmitting the bio-signals, a signal analysis processor for signal analysis, and a monitor for output to a screen and/or display and alarm. The present invention may also relate to a blood pressure management system that is convenient to be used and portable in the ubiquitous health and mobile health care environment.

### [Technical Solution]

According to an aspect of the present invention, there is provided a system for monitoring a blood pressure in real time, comprising a main body, and a sensor module including a first electrode, which is disposed on one surface of the main body and in contact with a human body when the main body is worn on the human body, and a second electrode, which is disposed on the other surface of the main body, to measure a signal relating to at least one of electrocardiogram (ECG), photoplethysmogram (PPG) and oxygen saturation (SpO₂) of the human body in contact with the first electrode and the second electrode.

In addition, other systems for implementing the present invention are further provided.

### [Advantageous Effects]

Continuously measuring a blood pressure may be achieved by applying a non-invasive method in a ubiquitous environment by using measurement electrodes provided integrally on front and rear surfaces of a wearable main body without a separate electrode configuration. Also, simultaneously measuring signals relating to electrocardiogram (ECG), photoplethysmogram (PPG), and oxygen saturation (Sp02) may be achieved among various bio-signals generated in a human body.

Further, increasing portability and convenience of use may also be achieved because a power supply is stably maintained and a bio-signal measured by using wireless communication may be transmitted or received. Furthermore, managing blood pressure of a hypertensive patient before a risk occurs may be achieved by continuously monitoring, and preventing high blood pressure for a normal person may be achieved.

### [Description of Drawings]

FIG. 1 is a block diagram schematically showing a configuration of the entire system according to the present invention.
FIGS. 2 and 3 are diagrams exemplarily showing an internal configuration of a blood pressure monitoring system according to an embodiment of the present invention.
FIGS. 4 to 6 are diagrams exemplarily showing a configuration of a wearable main body 203 included in a blood pressure monitoring system according to the embodiment of the present invention.

**< Descriptions of Reference Numerals >**

| | | | |
|---|---|---|---|
| 100: | communication network | 200: | blood pressure monitoring system |
| 201: | hardware block | 202: | software block |
| 203: | wearable main body | 210: | sensor module |
| 220: | gateway module | 230: | processor module |
| 240: | monitoring module | 250: | driver module |
| 300: | user terminal device | 400: | server |

### [Mode for Invention]

Embodiments of the present invention are described in detail below with reference to the accompanying drawings. The embodiments of the present invention are sufficiently described in detail such that those skilled in the art may carry out the present invention. It should be understood that although various embodiments of the present disclosure are different from each other, they need not be mutually exclusive. For example, in regard to an embodiment, specific forms, structures, and characteristics described herein may be realized through another embodiment without departing from the spirit and scope of the present invention. Moreover, it should be understood that locations or arrangements of separate elements within the disclosed embodiments may be changed without departing from the spirit and scope of the present invention. Accordingly, detailed descriptions which will be given below are not intended to be restrictive, and the scope of the present invention should be limited only by the accompanying claims and equivalents thereof. Similar reference numerals shown in the drawings denote elements performing identical or similar functions in several aspects.

Hereinafter, embodiments of the present invention will be described with reference to the accompanying drawings so that those skilled in the art to which the invention pertains may easily implement the invention.

### Configuration of the entire system

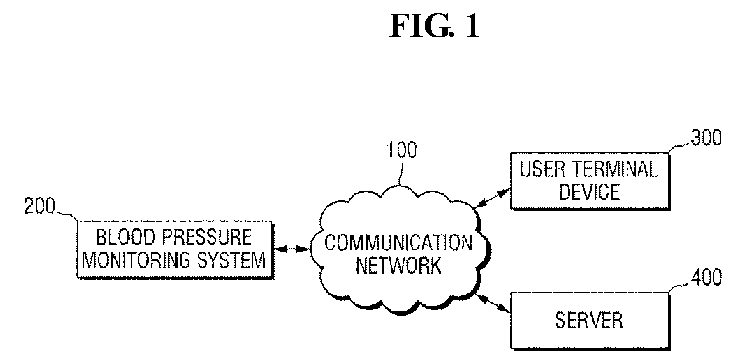

Hereinafter, wearable blood pressure monitoring systems and apparatuses according to an exemplary embodiment of the present invention will be described in detail. FIG. 1 is a block diagram schematically showing a configuration of an entire system. As shown in FIG. 1, the entire system may include a communication network 100, a blood pressure monitoring system 200, a remote user terminal device 300, and a remote server 400.

The communication network 100 is not necessarily limited to a particular communication method such as a wired or wireless communication method and may be implemented as various communication networks, such as a local area network (LAN), a metropolitan area network (MAN), and/or a wide area network (WAN). Examples of the communication network 100 may include LANs such as a Wireless-Fidelity (Wi-Fi) network, a Wi-Fi Direct network, a Long-Term Evolution (LTE) Direct network, and/or a Bluetooth network that are known in the art, but the present invention is in no way limited thereto. Thus the communication network 100 may at least partially include a typical wired/wireless communication network, a typical telephone network, and/or a typical wired/wireless television communication network.

The blood pressure monitoring system 200 may include a main body, a sensor including a first electrode that is disposed on one surface of the main body and in contact with a human body when the main body is worn on the human body, and a second electrode that is disposed on the other surface of the main body. The blood pressure monitoring system may be configured to serve to measure a signal relating to at least one of electrocardiogram (ECG), photoplethysmogram (PPG), and/or oxygen saturation (Sp02) of the human body in contact with the first electrode and the second electrode.

The blood pressure monitoring system 200 may include measurement electrodes on front and back surfaces of a wearable main body, and may include a sensor that may measure a signal relating to electrocardiogram (ECG), photoplethysmogram (PPG), and/or oxygen saturation (Sp02) of the human body. The sensor may digitally convert the signal and wirelessly transmit the signal. Further, the blood pressure monitoring system 200 may further include a gateway that receives bio-signal data wirelessly transmitted from the sensor and transmits the data to the Internet using IPv4 or IPv6-based TCP/IP communication.

Further, the blood pressure monitoring system 200 may further include a signal analyzing processor for signal analysis, a monitor for output to a screen and/or display and alarm, and a TCP/IP driver having an IPv4/IPv6 dual stack. Meanwhile, the blood pressure monitoring system 200 may implement Wireless Body Sensor Network (WBSN) technology which enables TCP/IP-based support for IPv6 with great reliability, ensuring connection to the internet and less vulnerability regarding security.

### Configuration of the blood pressure monitoring system

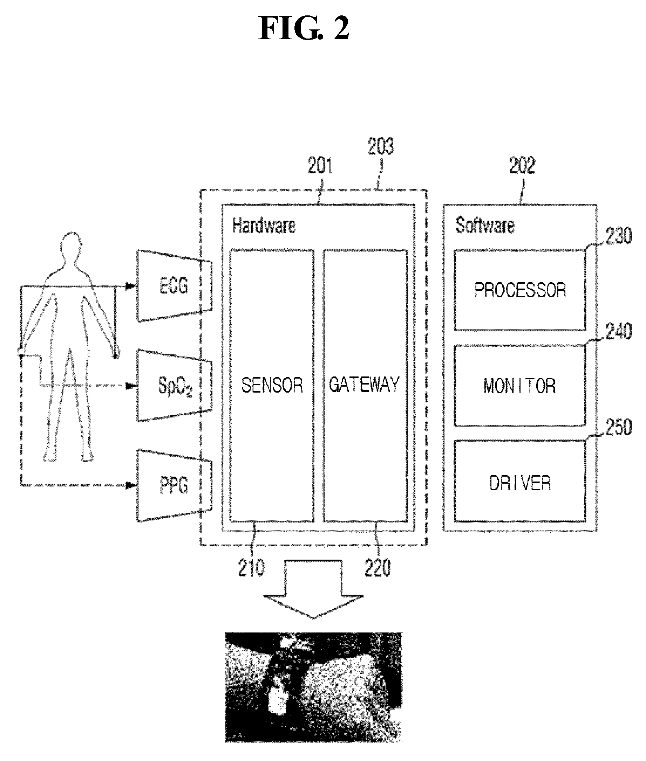

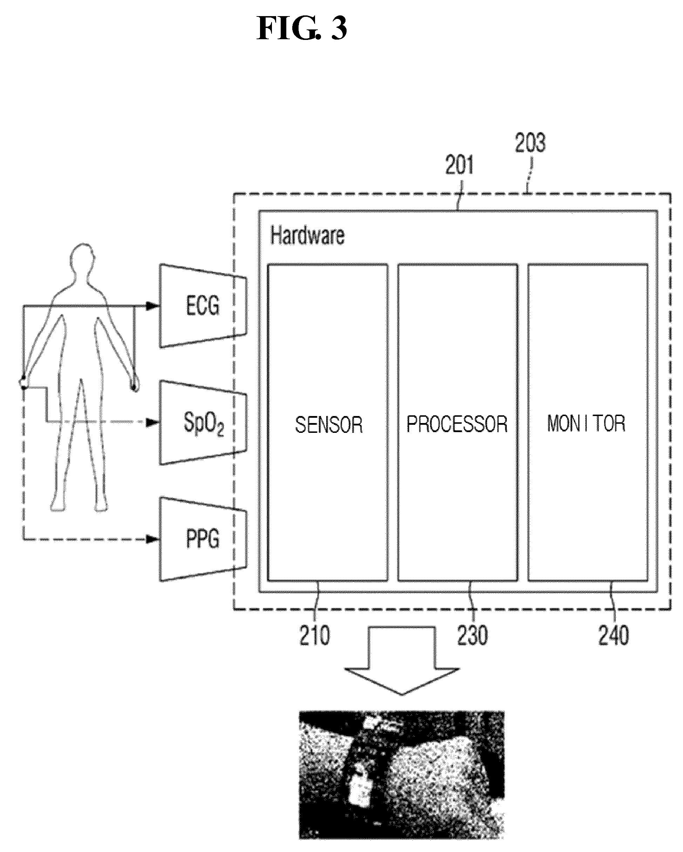

FIGS. 2 and 3 exemplarily show an internal configuration of the blood pressure monitoring system 200. Bio-signal measurement utilities operating based on the WBSN may be implemented based on an application operating in a local terminal device. Because data relating to the measured bio-signal may be vast, however, it may be difficult to implement a system useable by multiple users at the same time, in real time, and in a web-based environment. A blood pressure monitoring system thus may be provided that can operate in a Web-based environment and that may be applied to the analysis of a large amount of data.

Referring to FIG. 2, the blood pressure monitoring system 200 may include a hardware block 201 including a sensor 210 measuring a bio-signal and a gateway 220 transmitting the bio-signal. The blood pressure monitoring system 200 may also include a software block 202 including a signal analysis processor 230 for signal analysis, a monitor 230 for output to the screen and/or display and alarm, and a TCP/IP driver 250 having an IPv4/IPv6 dual stack.

Subsequently, referring to FIG. 2, a wearable main body 203 having measurement electrodes on the front and rear surfaces may include the sensor 210 and the gateway 220. Further, referring to FIG. 3, the wearable main body 203 having measurement electrodes on the front and rear surfaces may include the sensor 210 for measuring a bio-signal, the signal analysis processor 230 for signal analysis, and the monitor 240 for output to the screen and/or display and alarm.

Meanwhile, referring to FIG. 2, the sensor 210 of the blood pressure monitoring system 200 may continuously measure a variety of bio-signals, i.e., signals relating to electrocardiogram (ECG), photoplethysmogram (PPG), and oxygen saturation (Sp02), generated in the human body by applying a non-invasive method in a ubiquitous environment.

First, electrocardiogram (ECG) is a waveform including the vector sum of action potentials produced by the special excitatory and conductive system of the heart. An ECG signal is the vector sum of action potentials produced by various parts of the heart, such as a sinoatrial node (SA node), an atrioventricular node (AV node), His bundle, bundle branches, and Purkinje fibers, and may be measured by electrodes attached to the outside of the human body. For example, an ECG signal may be obtained by a standard limb lead method.

Next, a photoplethysmogram (PPG) signal is a pulse wave signal measured from the peripheral blood vessels when blood ejected from the heart during ventricular systole is delivered to the peripheral blood vessels. A PPG signal may be measured by using the optical properties of biological tissues. A PPG sensor, which is capable of measuring a pulse wave signal, may be attached to part of the human body such as the tip of a finger or toe where peripheral blood vessels are distributed, and the PPG sensor may measure the pulse wave signal after converting changes in the amount of blood flow, which are changes in the volume of the peripheral blood vessels, into changes in the amount of light.

Meanwhile, a PPG signal may not necessarily be used alone. Instead, information such as pulse transit time (PTT) information or pulse wave velocity (PWV) information may be extracted by analyzing the correlation between a PPG signal and an ECG signal, and may be used to diagnose cardiovascular disease. For example, a feature point may be obtained from the second derivative of a PPG signal, the interval between the feature point and the peak of (an R wave of) an ECG signal may be measured so as to extract a PTT signal and a PWV signal, and the PTT signal and the PWV signal may be used to diagnose the state of blood vessels, arteriosclerosis, peripheral circulation disturbance, etc.

An oxygen saturation (Sp02) signal is a bio-signal indicating the content of oxygen present in hemoglobin among various components of the blood.

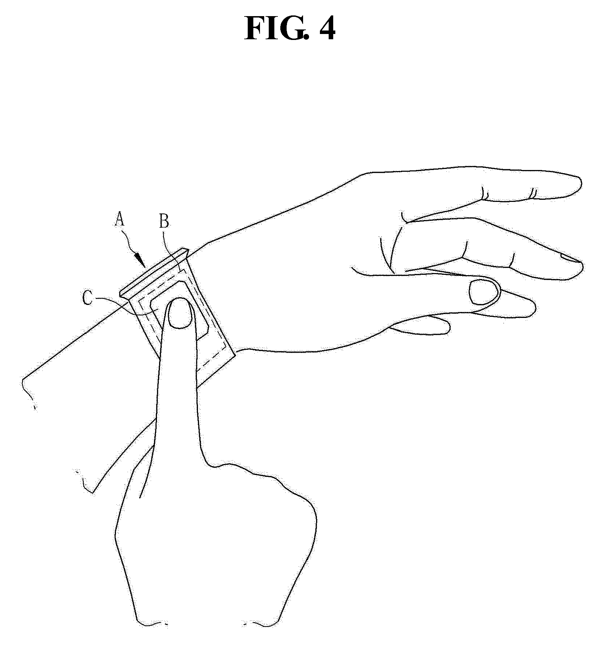

FIGS. 4 to 6 are diagrams exemplarily showing a configuration of the wearable main body 203 included in the blood pressure monitoring system. First, referring to FIG. 4, the wearable main body 203 included in the blood pressure monitoring system 200 may include a first electrode B disposed on the rear surface (for example an inside surface in contact with the wrist when the wearable main body 203 is worn on the wrist) of the wearable main body 203 to measure a bio-signal. The wearable main body 203 may include a second electrode C disposed on the front surface (for example, an outside surface not in contact with the wrist when the wearable main body 203 is worn on the wrist) of the wearable main body 203 to measure a bio-signal. After the user wears the wearable main body 203 on his/her wrist, if a body part of the user such as a finger contacts the second electrode C in a state where the first electrode B is in contact with the user's wrist, a signal relating to an electrocardiogram (ECG) of the user's body may be measured through the first electrode B and the second electrode C.

Subsequently, referring to FIG. 4, the second electrode C may be disposed on a display screen A of a monitor. In this case, as the user touches the display screen A on which the second electrode C is disposed, a signal relating to the electrocardiogram (ECG) of the human body may be measured through the first electrode B and the second electrode C. The display screen A may be formed of, for example, a touch screen.

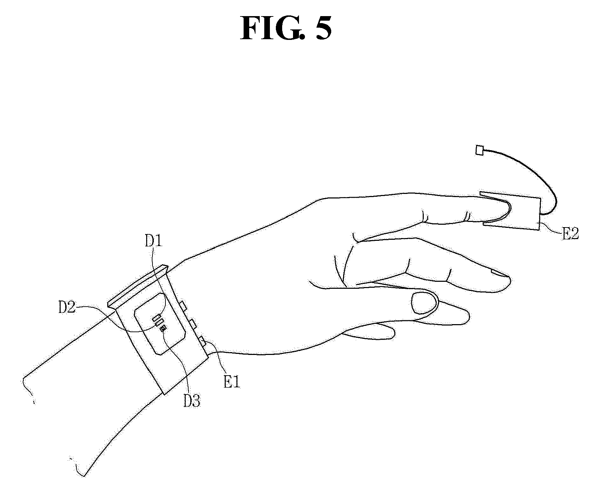

Referring to FIG. 5, the wearable main body 203 included in the blood pressure monitoring system 200 may include a connection terminal E1 that can be connected to a measurement processor capable of measuring a signal relating to photoplethysmogram (PPG) and oxygen saturation (Sp02). A measurement processor E2 capable of measuring a signal relating to a photoplethysmogram (PPG) and an oxygen saturation (Sp02) may measure a blood volume flowing in peripheral blood vessels of the tip of a finger or toe by irradiating red light to the tip of a finger or toe by using a red light source and measuring light reflected or transmitted from the human body by using a light sensor.

Referring to FIG. 5, the connection terminal E1 of the wearable main body 203 may be connected to a transmission type measurement processor E2, to which a fingertip is inserted, capable of measuring a signal relating to photoplethysmogram (PPG) and oxygen saturation (Sp02). The transmission type measurement processor E2 may include a light emitter (not shown) consisting of a red LED that generates light having a wavelength of about 660 nm and an infrared LED that generates light having a wavelength of about 940 nm. The measurement processor E2 may include a light receiver (not shown) consisting of an optical processor to which a photodiode and a phototransistor may be attached. The measurement processor and any other processors and/or modules and/or units and/or sensors and/or monitors herein may be operated by a controller having a memory and a processor

Referring to FIG. 5, the wearable main body 203 may include a reflective type measurement processor E2 capable of measuring a signal relating to photoplethysmogram (PPG) and oxygen saturation (Sp02). The reflective type measurement processor E2 included in the wearable main body 203 may include an LED D1 for generating red light of about 660 nm, an LED D2 for generating infrared light of about 940 nm, and an optical sensor D3 consisting of a photodiode or a phototransistor.

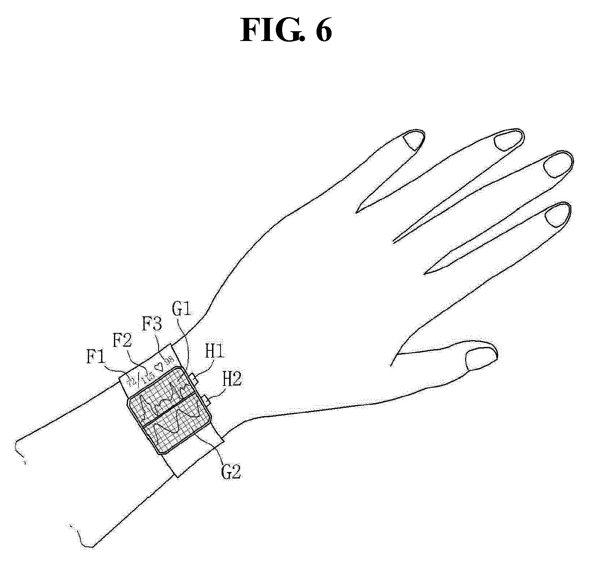

Referring to FIG. 6, a variety of information such as a systolic blood pressure F1, a diastolic blood pressure F2, and a heart rate F3 may be displayed on the display screen of the monitor 240 included in the wearable main body 203 included in the blood pressure monitoring system 200. In addition, a variation graph for an ECG signal G1 and a PPG signal G2 may be displayed on the display screen of the monitor 240. The display information or display style may be changed by a display mode selection button H1 or a display information selection button H2.

Meanwhile, a bio-signal measured by the wearable main body 203 of the blood pressure monitoring system 200 described above and data relating to the analysis results may be transmitted to the user terminal device 300 or the server 400 disposed outside through wireless communication such as Bluetooth or Wi-Fi provided in the wearable main body 203. Further, blood pressure may be estimated in real time based on a bio-signal measured by the wearable main body 203 of the blood pressure monitoring system 200 described above. For the details of a configuration for estimating the blood pressure on the basis of the bio-signal or a configuration for transmitting data on the bio-signal, reference can be made to Korean Patent Application Nos. 2013-116158 and 2013-116165, incorporated by reference herein in their entirety.

As described above, although the present invention has described specific matters such as concrete components, the embodiments and the drawings are provided merely to assist in a general understanding of the present invention, and the present invention is not limited to the embodiments. Various modifications and changes can be made from the description by those skilled in the art.

Accordingly, the spirit and scope of the present invention should not be limited or determined by the above-described embodiments, and it should be noted that not only the claims which will be described below but also their equivalents fall within the spirit and scope of the present invention.

## Claims

1. A system for monitoring a blood pressure in real time, comprising:
a main body; and
a sensor module including a first electrode, which is disposed on one surface of the main body and in contact with a human body when the main body is worn on the human body, and a second electrode, which is disposed on the other surface of the main body, to measure a signal relating to at least one of electrocardiogram (ECG), photoplethysmogram (PPG) and oxygen saturation (SpO₂) of the human body in contact with the first electrode and the second electrode.

2. The system of claim 1, wherein the sensor module includes at least one connection terminal which is connectable to at least one external measurement module.

3. The system of claim 2, wherein a measurement module for measuring a signal relating to at least one of photoplethysmogram (PPG) and oxygen saturation (SpO₂) is connected to the at least one connection terminal, and
wherein the measurement module includes a light emitting unit having at least one light emitting diode (LED) generating light to be irradiated to the human body and a light receiving unit having a photodiode (PD) detecting the light generated and transmitted through the human body.

4. The system of claim 1, wherein the main body includes a light emitting unit having at least one light emitting diode (LED) generating light to be irradiated to the human body and a light receiving unit having a photodiode (PD) detecting the light generated and reflected from the human body.

5. The system of claim 1, further comprising a gateway module which transmits information on a signal measured by the sensor module to an outside by using IPv4 or IPv6-based TCP/IP communication,
wherein the gateway module is incorporated in the main body.

6. The system of claim 1, further comprising a monitoring module which displays information on at least one of the measured signal and an analysis result of the measured signal,
wherein the monitoring module is incorporated in the main body.

7. The system of claim 6, wherein the second electrode is disposed on a display screen of the monitoring module to measure a signal relating to at least one of electrocardiogram (ECG), photoplethysmogram (PPG) and oxygen saturation (SpO₂) of the human body in contact with the display screen.

8. The system of claim 1, further comprising a processor module which estimates a blood pressure in real time by analyzing the measured signal,
wherein the processor module is incorporated in the main body.
